# EUROPEAN PATENT APPLICATION

(11) **EP 1 941 879 A1**
(43) Date of publication of application: **09.07.2008**
(21) Application number: 07000177.1
(22) Date of filing: 05.01.2007
(51) Int. Cl.: A61K 31/44, A61K 31/505, A61K 31/53, A61P 9/10

(54) **Neuroprotective agent for the treatment of neuronal damage**

(71) Applicant: PAION Deutschland GmbH, 52062 Aachen (DE)
(72) Inventor: Petersen, Karl-Uwe, 52072 Aachen (DE); Elger, Bernd, 13505 Berlin (DE); Brohman, Henning, 40589 Düsseldorf (DE); Oertelt, Clemens, 52062 Aachen (DE)
(74) Representative: Von Renesse, Dorothea

(57) **Abstract**

The use of a compound of the following general formula or a salt thereof is provided for the manufacture of a composition for the prevention or the treatment of a neurological damage in humans

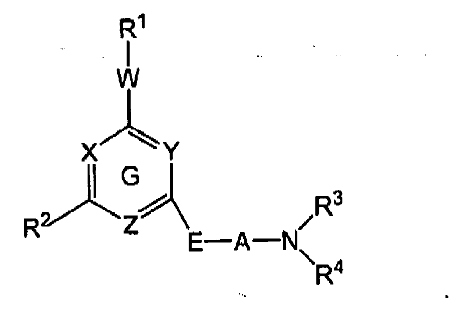

## Description

The invention relates to the field of neuroprotective treatment of stroke.

In the past a vast number of controlled clinical trials of acute stroke therapies have been published. However, only a few produced promising results to demonstrate that stroke is a treatable disorder in its hyperacute stage.

Even worse, neuroprotective drugs that aimed to salvage ischemic tissue, limit infarct size, prolong the time window for reperfusion therapy or minimise post-ischemic reperfusion injury or inflammation have shown great promise in preclinical testing but proved disappointing in clinical trials. More than 110 stroke trials have been performed in the past testing approximately 50 neuroprotective agents. However, none of the studied neuroprotective agents has proven successful clinically. Similarly, neuroprotective therapy has been unsuccessful in clinical trials of head trauma.

Due to this disillusioning balance it was acknowledged, that neuroprotection may never be effective for promoting functional recovery after brain injury in humans (David J. Gladstone, Sandra E. Black, Antoine M. Hakim, Stroke. (2002);33:2123-2136: Toward Wisdom From Failure. Lessons from Neuroprotective Stroke Trials and New Therapeutic Directions).

A variety of reasons have been inferred to explain the failure of neuroprotective agents to afford clinical benefit in ischemic stroke patients in the face of clear efficacy in preclinical experimental animal trials. A major reason is seen in the fact that available animal models do not adequately mimic ischemic stroke in humans. In particular, a problem in the evaluation of neuroprotective therapy is a tremendous variability of human stroke types, recovery patterns and/or associated clinical factors. Whereas e.g. preclinical studies usually involve middle cerebral artery occlusion in young healthy animals under anaesthesia with tightly controlled temperature, blood pressure, oxygenation and glucose levels, clinical trials often permit entry of multiple stroke types (cortical, mixed cortical-subcortical, pure subcortical, white matter stroke and in some neuroprotective trials, both ischemic and hemorrhagic stroke) and there is a lack of standardized control over physiological parameters. Unlike animal models, stroke patients typically have a multitude of associated variables that may affect prognosis, including different ages, comorbidities, polypharmacy, recurrent ischemia, poor collateral circulation or prior strokes. Hyperglycemia and other metabolic prognostic markers may be particularly important variables to control or adjust for in future trials (Gladstone et al.).

Thus, it is an objective of the present invention to provide a method for an effective clinical prevention or mitigation of neurological damage, namely an effective neuroprotective treatment. In particular it is an objective of the invention to provide means for treating, preventing or mitigating neuronal damage in patients or neuronal tissues with a reduced neuronal plasticity. In a particularly preferred embodiment the invention aims at providing means for treating elderly stroke patients.

According to the invention these objectives are accomplished by using a pharmaceutical composition comprising a compound of the following formula or a salt thereof as the active ingredient for the treatment of damaged neuronal tissues, in particular for the treatment of stroke (acute cerebral ischemia) in elderly human patients: 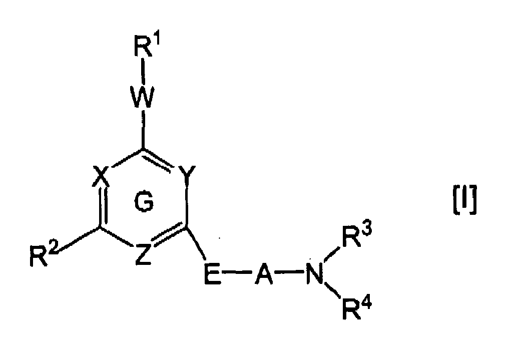
wherein R¹ represents an aryl group that may be substituted or a 5- through 10-membered heteroaromatic group that may be substituted, which is a monocyclic or fused ring system containing at least one hetero-atom selected from the group consisting of nitrogen, oxygen, and sulphur as a ring member, said aryl group and heteroaromatic group may be respectively substituted by 1-3 substitutes, whether the same or different, as selected from the group consisting of hydroxy, halogen, alkyl, haloalkyl, hydroxyalkyl, aralkyl, alkenyl, alkoxy, haloalkyloxy, alkylthio, cycloalkyl, cycloalkylalkyl, cycloalkyloxy, alkylsulfonyl, sulfamoyl, alkanoyl, amino, monoal-kylamino, dialkylamino, carboxy, alkoxycarbonyl, cyano, and nitro;
R² represents hydrogen, alkyl, alkenyl, cycloalkyl, cycloalkylalkyl, hydroxyalkyl, haloalkyl, alkoxy, alkylthio, amino, monoalkylamino, dialkylamino, or phenyl that may be substituted by 1-3 same or different substitutes selected from the group consisting of halogen, alkyl, and alkoxy;
R³ and R⁴ may be the same or different and each represents hydrogen or alkyl that may be substituted by one or more substitutes selected from the group consisting of hydroxy, alkoxy, amino, monoalkylamino, and dialkylamino, or R³ and R⁴ taken together with the adjacent N atom represent a 4- through 8-membered cyclic amino group of the formula NR³R⁴, which may have N, O, or S in addition to said N atom as a ring member and may be substituted by one or more substitutes selected from the group consisting of alkyl, alkoxy, hydroxy, oxo, amino, monoalkylamino, dialkylamino, aryl that may be substituted, and pyridyl that may be substituted;

A represents alkylene of 2-10 carbon atoms, which may be substituted by 1 or 2 same or different substitutes selected from the group consisting of alkoxy, hydroxy, and oxo in optional substitutable positions:
E represents O or S;
W represents a single bond, O, S, or (CH₂)ₙ, wherein CH₂ may be substituted by alkyl; n is an integer of 1 or 2;
X, Y, and Z may be the same or different and each represents CH, CR (where R represents alkyl), or N; excluded, however, is the case in which X, Y, and Z concurrently represent carbon; thus, ring G represents pyridine, pyrimidine, or 1,3,5-triazine;
when 1-3 of X, Y, and Z represent N, one of them may form an oxide.

This class of compounds is subject of the European patent EP 0 781 766 B1 (corresponding to the US 5,945,426), which is fully incorporated herewith by references.

In a preferred embodiment R¹ represents halogen-substituted phenyl; R² represents alkyl or holoalkyl; -NR³R⁴ represents a 4-through 8-membered cyclic amino group containing only one nitrogen atom as a ring-constituting hetero-atom; A represents alkylene of 3-6 carbon atoms; E represents O or S; W represents a single bond; X and Z respectively represent N with Y representing CH or Z represents N with X and Y respectively representing CH.

In a further embodiment NR³R⁴ represent piperidino; A represents alkylene of 4-6 carbon atoms; E represents O; W represents a single bond; and X and Z respectively represent N with Y representing CH or Z represents N with X and Y respectively representing CH.

In yet another embodiment the compound is selected from the group consisting of
4-(4-fluorophenyl)-2-methyl-6-(4-piperidinobutoxy)pyrimidine,
4-(4-fluorophenyl)-2-methyl-6-(5-piperidinopentyloxy)pyrimidine,
4-(4-fluorophenyl)-2-methyl-6-(6-piperidinohexyloxy)pyrimidine,
4-(4-fluorophenyl)-2-methyl-6-(1-methyl-4-piperidinobutoxy)pyrimidine,
2-(4-fluorophenyl)-4-methyl-6-(4-piperidinobutoxy)pyrimidine,
4-(4-fluorophenyl)-2-methyl-6-(3-piperidinopropoxy)pyridine, and
4-(4-fluorophenyl)-2-methyl-6-(5-piperidinopentyloxy)pyridine, or a salt thereof, or a solvate thereof.

The particularly preferred compound is 4-(4-fluorophenyl)-2-methyl-6-(5-piperidinopentyloxy)pyrimidine, a synonym of which is Enecadin or NS-7. This compound is preferably used as its hydrochloride salt.

Compounds of the above general formula are referred to hereinafter as the "neuroprotective agent".

According to the invention the neuroprotective agent is in particular used for treating stroke in patients with a reduced neuronal plasticity compared to healthy young adults. A reduction in neuronal plasticity is frequently concomitant with an impaired capacity of functional recovery. Both properties are especially characteristic for neuronal tissues of people in advanced age. Thus, one particular target group of the method of the present invention are elderly people.

The term "elderly people" as used in the present invention refers to a group of patients of at least 55 years, more preferred of at least 65 and most preferred of at least 70 years. Thus, in one embodiment, the method according to the invention relates to the treatment of neurological tissues in patients of at least 55 years.

As outlined above, in one embodiment the inventions enables the treatment of stroke patients who are characterized by having a brain which differs substantially compared to the brains of younger patients in terms of physiology, neuronal chemistry and/or pathophysiological features. For example, advancing age of humans and animals is associated with abnormalities in glycolytic flux, lactate production, oxidation and energy production (Jin, K., Minami, M., Xie, L., Sun, Y., Mao, X.O., Simon, R.P., Greenberg, D.A.; Aging Cell 3, 373-377, 2004; Darsalia V., Heldmann, U., Lindvall, O., Kokaia Z.; Stroke 36(8), 1790-1795, 2005). In particular, it is known. that the aging brain displays reduced neuronal plasticity and thus exhibits a poor functional recovery after stroke compared to younger brains. This age-related reduction of neuronal plasticity is *inter alia* accompanied by a reduction in the upregulation of factors promoting brain plasticity, such as microtubule-associated protein 1B (MAB1B). and beta-amyloid precursor protein (betaAPP). Additionally, reduction of adenylate cyclase forms of neuronal activity, especially in the hippocampus can be observed in aged brains (Reis G.F., Lee, M.B., Huang A.S., Parfitt K.D-; J Neurophysiol 93(6) 3381-9, 2005). Simultaneously, the glial reactivity and the accumulation of Abeta, a proteolytic cleavage product of betaAPP with neurotoxic properties increase (Badan I, Platt D, Kessler C, Popa-Wagner A., Gerontology. 2003 49(6):356-65: Temporal dynamics of degenerative and regenerative events associated with cerebral ischemia in aged rats). Additionally, brain morphology of aged patients displays different features compared to younger patients. It has been shown that a reduced number of neurons is detectable in aged brains (Sowell ER, Thompson PM, Toga AW. Mapping changes in the human cortex throughout the span of life. Neuroscientist 10: 372-392 (2004); Buckner RW. Memory and executive function in aging and AD: Multiple factors that cause decline and reserve factors that compensate. Neuron 44: 195-208 (2404)), as well as the occurrence of cortical atrophy. Animal models could show that a characteristic for aged brain is additionally attenuated NMDA-mediated responses (Gonzales, RA, Brown LM, Jones TW, Trent RD, Westbrook SL, Leslie SW, Neurology of Aging, 12, 219-225, 1991).

Another property of aging is a significant loss of gray matter volume in the brain. Global gray matter volume significantly declines with age, and regional patterns suggest that above and beyond the global gray matter loss, gray matter density reduction occurred in regions including bilateral superior parietal regions and other specific locations.

The morphological changes that occur throughout aging of humans can be evaluated by visualization techniques, which are known to the ordinary skilled person. In particular, non invasive imaging tools such as magnetic resonance imaging (MRI) or positron emission tomography (PET) can be applied. Using MRI, thus, provides a tool for understanding when "maturation" of the brain stops and "aging" starts. Accordingly, MRI techniques allow discriminating between mature and "aged" brains. Details about the MRI techniques used for the evaluation of maturation versus aging are given in the review of Sowell et al. (Neuroscientist 10(4): 372-392, 2004). This review is incorporated herein by reference for the disclosure of morphological changes of aged brains and the clinical evaluation thereof by MRI technology. Consequently, visual rating scales were developed that correlate white matter changes in MRI with age (Pantoni L., Simoni M., Pracucci G., Schmidt R., Barkhof F. and Inzitari D., Stroke, 33: 2827-2833, 2002).

Another visualization technique - PET - has been utilized to identify changes in cerebral blood flow (CBF) and a high correleation between age and decline on CBF was found (Silverman and Phelps, Mol Genet Metab 74:128-138, 2001).

Thus, in a preferred embodiment of the invention patients are treated with the neuroprotective agent which are characterized by an aged brain as identifiable with non invasive imaging tools such as MRI or others.

In an advantageous embodiment of the invention, the neuroprotective agent is administered to the patient immediately after stroke onset or within six, twelve, or seventy-two hours after the beginning of the stroke.

### I, Animal Trials

### 1.1. Animals

The experiments have been performed in accordance to the requirements of the German laws for the protection of animals (Deutsches Tierschutzgesetz) and in compliance with the European Community directive 86/609. The animals were maintained under standard conditions (12 h day/night cycle, 22 °C, 50 % humidity) with free access to food and tap water. Before use for the experiments, the animals were allowed to recover from transportation for about one week.

### 1.2. Permanent MCA occlusion in rats

Male Fischer-344 rats which were either young adult (3 months, body weights: 250-300 g) or aged (21 months, body weights: 410-480 g) were used for permanent MCA occlusion as described in detail elsewhere (Elger et al., 2004). Briefly, animals were anaesthetised with 4 % halothane for induction and 1.5% halothane for maintenance in 30% O₂/70% N₂O via a face mask. After a vertical 2 cm skin incision was made between the left eye and the left ear, the temporalis muscle was partly removed in order to expose the zygoma and the squamosal bone. The left MCA was exposed through a burr hole craniectomy (2 mm diameter) drilled in close proximity to the foramen ovale. After opening the dura, the proximal portion of the MCA was electrocauterised with bipolar forceps (Erbotom T130, Erbe, Elektromedizin GmbH, Tübingen, Germany) and cut through with scissors. The burr hole was covered with Lyostypt (Braun Dexon) and the skin was sutured. During surgery which did not exceed 10 minutes, the body temperature of the anaesthetised animals was maintained at 37 ± 05, °C by a feedback regulated heating pad.

### 1.3. Permanent MCA occlusion in mice

Male mice (NMRI strain) weighing 23-30 g were anaesthetised with Avertin (tribromoethanol) dissolved in 10% cremophor), 240 mg/kg i.p. in a volume of 10 ml/kg. The distal portion of the MCA was electrocoagulated with a bipolar diathermy above the level of the olfactory tract as described in detail elsewhere), Briefly, a skin incision was made vertically on the left side between the ear and the orbit, parts of the underlying temporal muscle were removed and a small hole (1 mm) was made into the skull. After electrocoagulation of the MCA, the wound was sutured. The animals did not sustain blood loss, and all surgeries were completed within 7-10 min.

### 1.4. Measurement of lesion volume using 2,3,5-triphenyltetrazolium chloride (TTC)

Total infarct volume was determined in all rats and mice 48 hours after MCA occlusion as described before. Briefly, brains of anaesthetised animals were perfused in situ with a saline solution containing 10% TTC. After storage in fixative (4% paraformaldehyde and 2% sucrose in 0,1 M sodium phosphate) for three days, the brains were sliced and infarct volumes were calculated using a computerised image analysis system.

### 1.5. Treatment paradigms

All rats and mice received a post-treatment consisting of a single i.v. injection of vehicle solution (0.9% NaCl) or NS-7 after MCA occlusion. In first experiments, young adult rats (3 months of age) were treated with NS-7 at dosages of 0.1 or 0.3 mglkg immediately after MCA occlusion. In parallel, mice were used as screening tool (Backhauss et al., 27, 27-32, 1992 and treated in the same way as the rats for comparison of the drug effects in two different species. In a second series of experiments, delayed administration (6 or 12 h) of NS-7 was tested in young adult rats using again the dosages of 0.1 or 0.3 mg/kg. Finally, the effect of NS-7 was investigated in aged rats (21 months) using the most efficient dosage (0.3 mg/kg) and the most delayed time window (6 h) that had shown significant cerebroprotection in the second series of experiments.

### 1.6. Statistical analyses

All results are given as mean and standard deviation (S.D,). For each experimental group the deviation from the Gaussian distribution was tested by the Kolmogorov-Smimov test. All the data passed the normality test. Statistical comparisons between control and treatment groups were made using analysis of variance followed by post-hoc Dunnett's multiple comparison test.

### 2. Results

### 2.1. Comparison of NS-7 after permanent focal cerebral ischemia in rats and mice

Rats and mice were given a single i.v. injection of NS-7 (0.1 or 0.3 mg/kg) or vehicle (0.9% NaCl) immediately after permanent MCA occlusion and infarct volumes were histologically assessed 48 h later. Both cerebral cortex and striatum were affected by ischemia on the ipsilateral side after proximal MCA occlusion in rats. Following distal MCA occlusion in mice, the ischemic territory was strictly ipsi-lateral (left side) and localised exclusively in the temporo-parietal cortex.

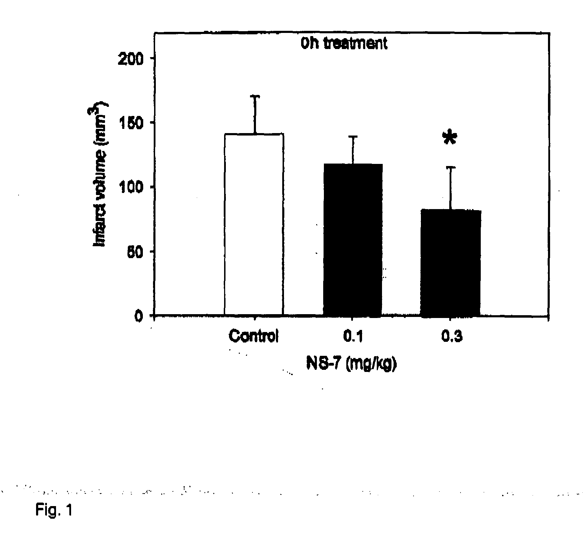

In rats, NS-7 reduced total infarct volume dose-dependently from 141 ± 29 mm³ in vehicle-treated controls (n=8) to 117 ± 23 mm³ and 82 ± 34 mm³ (p<0,05) in the low dosage group (n=8) and the higher dosage group (n=10), respectively (Fig. 1). In mice, infarct volumes were diminished by NS-7 as summarised in table 1. On average, the higher dose of NS-7 produced a reduction of total infarct volume by 42% in rats and by 37% in mice.

### 2.2. Time window of NS-7 administration after permanent focal cerebral ischemia in rats

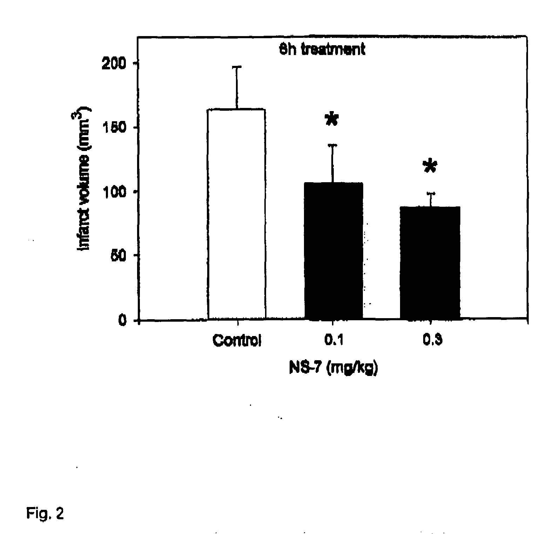

Dose-dependent reductions of total infarct volume were observed in rats administered a single intravenous injection of NS-7 six hours after MCA occlusion (Fig. 2). Infarct volumes were 164 ± 33 mm³ in vehicle-treated controls (N=8), 106 ± 29 mm³ in rats (n=6) given 0,1 mg/kg NS-7 and 87 ± 11 mm³ in rats (n=6) given 0.3 mg/kg NS-7. However, NS-7 injection 12 h after MCA occlusion had no effect as the infarct volumes were 136 ± 71 mm³ in vehicle-treated controls (n=8), 148 ± 60 mm³ in rats (n=9) given 0.1 mg/kg NS-7 and 119 ± 41 mm³ in rats (n=11) given 0.3 mg/kg NS-7.

### 2.3. Effects of 6 h delayed therapy with NS-7 in aged rats

In aged rats (21 months), cerebral infarct volume was reduced by 41% from 124 ± 63 mm³ in controls (physiological saline, n=7) to 73 ± 55 mm³ in rats (n=9) given a single i.v. injection of NS-7 (0,3 mg/kg) 6 h after MCA occlusion. The magnitude of effect of 6 hour delayed NS-7 therapy in aged rats was almost as pronounced as in young adult rats (47%), however, it was statistically not significant due to a 2-fold higher standard deviation of infarct volumes in aged rats which affords larger numbers of animals per group to reach statistical significance.

**Table 1 Effect of NS-7 on total infarct volume in mice measured 48 h after permanent MCA occlusion (mean ± S.D.). Animals were given a single i.v. injection of vehicle solution (0,9% NaCl) or NS-7 immediately after MCA occlusion.**

| Groups | Treatment | N | Total infarct volume (mm³) |
|---|---|---|---|
| 1 | Vehicle | 15 | 25.1 ± 9.0 |
| 2 | NS-7 (0.1 mg/kg) | 16 | 23.8 ± 6.2 |
| 4 | NS-7 (0-3 mg/kg) | 16 | 15.7 ± 5.6 * |

| | | | |
|---|---|---|---|
| * p<0,05 vs. group 1 | | | |

### LEGEND TO FIGURES

- Fig. 1: Effect of the Na⁺/Ca²⁺ channel blocker NS-7 on total infarct volume in rats measured 48 h after permanent MCA occlusion (mean ± S.D., p<0.05 vs. controls). Treatment with NS-7 consisted of a single i.v. injection given immediately after MCA occlusion.
- Fig. 2: Effect of NS-7 on total infarct volume in rats measured 48 h after permanent MCA occlusion (mean ± S.D., p<0.05 vs. controls). A single i.v. injection of NS-7 was administered 6 h after MCA occlusion.

## Claims

1. The use of a compound of the following general formula or a salt thereof for the manufacture of a composition for the prevention or the treatment of a neurological damage in humans 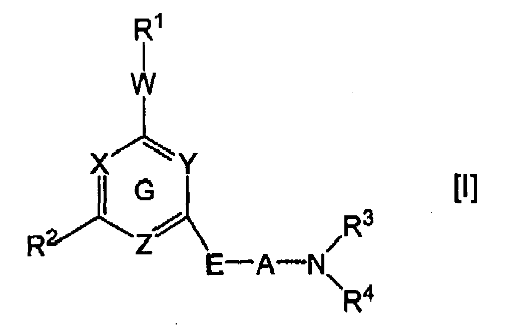
wherein R¹ represents an aryl group that may be substituted or a 5- through 10-membered heteroaromatic group that may be substituted, which is a monocyclic or fused ring system containing at least one hetero-atom selected from the group consisting of nitrogen, oxygen, and sulfur as a ring member; said aryl group and heteroaromatic group may be respectively substituted by 1-3 substitutes, whether the same or different, as selected from the group consisting of hydroxy, halogen, alkyl, haloalkyl, hydroxyalkyl, aralkyl, alkenyl, alkoxy, haloalkyloxy, alkylthio, cycloalkyl, cycloalkylalkyl, cycloalkyloxy, alkylsulfonyl, sulfamoyl, alkanoyl, amino, monoalkylamino, dialkylamino, carboxy, alkoxycarbonyl, cyano, and nitro;
R² represents hydrogen, alkyl, alkenyl, cycloalkyl, cycloalkylalkyl, hydroxyalkyl, haloalkyl, alkoxy, alkylthio, amino, monoalkylamino, dialkylamino, or phenyl that may be substituted by 1-3 same or different substitutes selected from the group consisting of halogen, alkyl, and alkoxy;
R³ and R⁴ may be the same or different and each represents hydrogen or alkyl that may be substituted by one or more substitutes selected from the group consisting of hydroxy, alkoxy, amino, monoalkylamino, and dialkylamino, or R³ and R⁴ taken together with the adjacent N atom represent a 4- through 8-membered cyclic amino group of the formula NR³R⁴, which may have N, O, or S in addition to said N atom as a ring member and may be substituted by one or more substitutes selected from the group consisting of alkyl, alkoxy, hydroxy, oxo, amino, monoalkylamino, dialkylamino, aryl that may be substituted, and pyridyl that may be substituted;
A represents alkylene of 2-10 carbon atoms, which may be substituted by 1 or 2 same or different substitutes selected from the group consisting of alkoxy, hydroxy, and oxo in optional substitutable positions;
E represents O or S;
W represents a single bond, O, S, or (CH₂)ₙ, wherein CH₂ may be substituted by alkyl; n is an integer of 1 or 2;
X, Y, and Z may be the same or different and each represents CH, CR (where R represents alkyl), or N; excluded, however, is the case in which X, Y, and Z concurrently represent carbon; thus, ring G represents pyridine, pyrimidine, or 1,3,5-triazine;
when 1-3 of X, Y, and Z represent N, one of them may form an oxide.

2. The use according to claim 1 wherein the humans are patients with a significantly reduced neuronal plasticity.

3. The use according to claim 2, wherein the patients have a significantly reduced volume of gray matter.

4. The use according to claim 2 or 3, wherein the patients have at least 55 years, preferably at least 60, more preferably at least 65 or at least 70 years.

5. The use according to one of the above claims, wherein the patients are elderly stroke patients.

6. The use according to one of the above claims, wherein
R¹ represents halogen-substituted phenyl;
R² represents alkyl or haloalkyl;
NR³R⁴ represents a 4-through 8-membered cyclic amino group containing only one nitrogen atom as a ring-constituting hetero-atom;
A represents alkylene of 3-6 carbon atoms;
**E represents O or S;**
W represents a single bond;
X and Z respectively represent N with Y representing CH or Z represents N with X and Y respectively representing CH.

7. The use according to one of the above claims wherein
NR³R⁴ represent piperidino;
A represents alkylene of 4-6 carbon atoms;
E represents O;
W represents a single bond; and X and Z respectively represent N with Y representing CH or Z represents N with X and Y respectively representing CH.

8. The use according to any of the above claims, wherein the active ingredient is a compound selected from the group consisting of
4-(4-fluorophenyl)-2-methyl-6-(4-piperidinobutoxy)pyrimidine,
4-(4-fluorophenyl)-2-methyl-6-(5-piperidinopentyloxy)pyrimidine,
4-(4-fluorophenyl)-2-methyl-6-(6-piperidinohexyloxy)pyrimidine,
4-(4-fluorophenyl)-2-methyl-6-(1-methyl-4-piperidinobutoxy)pyrimidine,
2-(4-fluorophenyl)-4-methyl-6-(4-piperidinobutoxy)pyrimidine,
4-(4-fluorophenyl)-2-methyl-6-(3-piperidinopropoxy)pyridine and
4-(4-fluorophenyl)-2-methyl-6-(5-piperidinopentyloxy)pyridine, or a salt thereof,
or a solvate thereof.

9. The use according to any of the above claims wherein the patients are selected from the group of humans of at least 55 years, preferably of at least 65 years, most preferred of at least 70 years.

10. The use according to any of the above claims wherein humans each have a brain of reduced neuronal plasticity and/or reduced neurogenesis potential.
